# EUROPEAN PATENT SPECIFICATION

(11) **EP 2 053 917 B1**
(45) Date of publication and mention of the grant of the patent: **03.03.2010**
(21) Application number: 07787926.0
(22) Date of filing: 26.07.2007
(51) Int. Cl.: A01N 37/52, A01N 43/56

(54) **COMPOSITION FOR ENHANCED ANTIPARASITIC ACTIVITY**
ZUSAMMENSETZUNG FÜR VERSTÄRKTE ANTIPARASITISCHE WIRKSAMKEIT
COMPOSITION À ACTIVITÉ ANTIPARASITAIRE AMÉLIORÉE

(30) Priority: 08.08.2006 EP 06118620; 08.08.2006 US 836523 P
(43) Date of publication of application: 06.05.2009
(73) Proprietor: Intervet International BV, 5831 AN Boxmeer (NL)
(72) Inventor: WILLIAMS, Heike, 55270 Schwabenheim (DE); MERTENS, Christina, NL-5831 AN Boxmeer (NL); MICULKA, Christian, 65195 Wiesbaden (DE); PFLUGSEDER, Karin, 55270 Schwabenheim (DE); STREBER, Wolfgang, 55270 Schwabenheim (DE)
(74) Representative: Stumm, Karin
(86) International application number: PCT/EP2007/057700
(87) International publication number: WO 2008/017591

(56) References cited:
- US-B2- 6 543 389

## Description

The present invention relates to veterinary compositions for the control of parasitic acarids and their use for the manufacture of a veterinary medicament for the control of ectoparasite infestations on animals.

A number of pests and parasites can infest or infect domestic animals such as cattle, horses, pigs, sheep and also companion animals such as cats and dogs. These pests and parasites are of great nuisance to both the animals and their owners. External parasites such as ticks, mites, lice, e.g. chewing lice, flies, e.g. keds and fleas irritate the animals and can cause disease, either by themselves, or by carrying vector transmitted pathogens.

Ticks are important blood feeding arthropod parasites that belong together with mites (Family: *Psoroptidae*) to the order *Acarina*. There are two well-established families of ticks, the *Ixodidae* (hard ticks), and *Argasidae* (soft ticks). The *Ixodidae* species is of most economic importance and include the important species *Boophilus* spp., *Rhipicephalus* spp, *Ixodes* spp, *Hyalomma* spp., *Amblyomma* spp. and *Dermacentor* spp.

Ticks produce injury after infestation of animals in three respects: direct damage caused by parasitism such as local injury and blood loss; by toxins injected by the parasites and by the transmission of diseases. Especially in companion animals, ticks may be the source of tick transmitted diseases.

Mange is a chronic skin diseases of mammals caused by parasitic mites and characterized by skin lesions, itching, and loss of hair

Amitraz is a known agricultural and veterinary insecticide and acaricide and is e.g. commercially available in the products Taktic ®, Topline ®, Ectodex ® (Intervet International bv, Boxmeer, The Netherlands). Amitraz is a valuable veterinary product effective against strains of ticks resistant to other chemical classes of ixodicides. It also possesses sufficient persistence on hair and wool to control all stages of parasitic ticks. The unique expellent action of amitraz causes ticks to withdraw mouthparts rapidly from, and fall off, the host animal.

Atipamezole is a selective alpha₂ - adrenoceptor antagonist which is currently marketed under the trademark Antisedan® for the reversal of sedative-analgesic veterinary drugs. Atipamezole has been disclosed e.g. in the European Patent EP 183492 as useful for the reversal of detomidine. European Patent EP 0589957 discloses the use of atipamezole for the treatment of male sexual impotence. In US 4698692 the use of atipamezole for the attenuation of ethyl alcohol intoxication is disclosed.

US Patent No. US6543389 discloses insecticidal pet collars for dogs comprising amitraz and atipamezole. Atipamezole in the collar provides amelioration of amitraz toxicosis in combination with the amitraz in case the dogs ingests the collar. The pet collar comprises 0.01 to 1%, preferably 0.1 to 1%, by weight of atipamezole.

Safe, effective ways to eliminate ectoparasites are desired for the companion animal's well-being, for the well-being and comfort of its human associate and for the prevention of losses in livestock

The current invention relates to a veterinary composition comprising amitraz and atipamezole or its pharmaceutically acceptable salt with improved activity against parasites compared to amitraz alone. The composition is particularly suitable for dermal control of parasitic acarids, particularly ticks on mammals, lice as well, as premise control of susceptible acarids and insects.

It has been found that the combination of amitraz and atipamezole provides an improved acaricidal effect compared to the effects of amitraz. Surprisingly the addition of atipamezole significantly enhanced the activity of amitraz against ectoparasites and especially acarides and thus provides excellent control of parasitic acarides and/or insects. This is an especially unexpected finding because atipamezole alone does not show an acaricidal/insecticidal effect.

The term "atipamezole" as used herein means 4(5)-(2,3-dihydro-2-ethyl-1H-inden-2-yl)imidazole. A method for the preparation of atipamezole is provided for in U.S. Pat. No. 4,689,339.

The term "pharmaceutically acceptable salt" as used herein includes acid addition salts, hydrates, alcolates and other salts which are physiologically compatible in animals. The acid addition salts may be formed by either strong or weak acids. Representative of strong acids are hydrochloric, sulfuric and phosphoric acids. Representative of weak acids are fumaric, maleic, succinic, oxalic, citric, tartaric, cyclohexamic and the like.

The term "atipamezole hydrochloride" means the hydrochloride acid addition salt of atipamezole.

Amitraz has the chemical name 1,5-di-(2,4-dimethylphenyl)-3-methyl-1, 3, 5-triazapenta-1,4-diene, and an IUPAC name of N-methylbis(2,4-xylyliminomethyl)amine. Its synthesis is described in GB 1,327,935. Amitraz presents adrenergic activity by monoaminooxidasis inhibition, leading to noradrenalin and serotonin at central nervous system.

Preferably the combination of active ingredients according to the invention is administered topically. Examples of topical administrations suitable for use in the method of the invention include spot-on, pour-on, dip, wash, shampoo, foam, gel, spray, lotion, powder, collar, flour or any of the conventional means of topically applying a veterinary composition. The topical mode or administration will vary with the species and size of the host animal.

The ratio in the veterinary antiparasitic composition of the invention is 8 weight part of amitraz to 1 weight parts of atipamezole to 1:10, preferably 4:1 to 1:4. In one embodiment the ratio is 1:2

In one embodiment the invention relates to a liquid veterinary composition comprising amitraz and atipamezole or its pharmaceutically acceptable salt.

Various techniques for administration of liquid veterinary compositions to animals are available. Preferably the combination of active ingredients according to the invention is administered topically. Liquid compositions for the control of ectoparasites can be e.g. applied topically as bath treatment, dip, spray, shampoo, foam, gel or lotion, as pour-on or spot-on. Ear ticks may be controlled by composition applied directly into the ears.

For the preparation of the liquid veterinary composition of the invention for such administration, it is suitable to formulate the composition e.g. as a wettable powder, aqueous concentrate, emulsifiable concentrate (EC), liquid concentrate, sol (flowable agent), aerosol, or the like, by conventional methods such as admixing the compounds with suitable carriers and auxilliaries.

As suitable carrier and auxilliaries, there may be mentioned liquid carriers, emulsifiers, suspending agents, dispersants, extenders, penetrating agents, wetting agents, thickeners, defoaming agents, stabilizers or antifreezing agents. They may be added as the case requires. Such carriers and auxiliaries are known in the art e.g. as described in "Gennaro, Remington: The Science and Practice of Pharmacy" (20th Edition, 2000) incorporated by reference herein. All such components, carriers and excipients must be substantially pharmaceutically or veterinary pure and non-toxic in the amounts employed and must be compatible with the active ingredients.

The composition may be formulated for application by a particular method, for example spraying, in which case the formulation may be presented as an aerosol using a liquid or gas propellant. For the administration as pour- on or spot-on suitable components and carriers for localized dermal administration are included in the composition.

The ratio in the liquid antiparasitic composition of the invention is 100 weight part of amitraz to 1 weight parts of atipamezole to 1:10, or 4:1 to 1:4. or 1:2 to 2:1.

In commercially useful formulations, the composition of the invention may also be present in a mixture with other active agents, for example various insecticidal, or acaricidal agents in order to expand its applicability. Other ingredients that may be included in the composition of the current invention are pesticidial active ingredients e.g. synthetic pyrethroids (e.g permethrin, deltamethrin, cypermethrin, lambdacyhalothrin, fenvalerate, resmethrin, tralomethrin, cyphenotrin), acetylcholinesterase Inhibitors as carbamates (e.g. carbaryl, benziocarb, fenoxycarb, proxopur), acetylcholine mimics (e.g. nicotine, imidacloprid) neonicotinoid compounds, e.g. nitenpyram, GABA Antagonists (e.g. fipronil).

In another embodiment the invention relates to the use of the composition according to the invention for the manufacture of a veterinary medicament for the control of parasitic insect- and acarid- infestations on animals.

The use of atipamezole or its pharmaceutically acceptable salt improves the antiparasitic activity especially the acaricidal activity of amitraz.

Specifically, the use of atipamezole or its pharmaceutically acceptable salt improves the detachment effect of amitraz on ticks.

In the composition according to the invention the active ingredients are present in the dosage form as true mixtures, but they could also be administered individually in separate dosage forms and form mixtures only on the host organism.

In case amitraz and atipamezole are administered individually in separate dosage forms they are administered in parallel. Parallel means that the active ingredients may be administered at the same time, that is simultaneously, but they may also be administered sequentially, that is one after the other i.e. so that they are present together for certain periods at the latest when they are taken up by the parasite so that the desired effect arises.

The active ingredients are preferably administered simultaneously. When given simultaneously the composition according to the invention is preferably presented as a single dosage form comprising both amitraz and atipamezole in a single formulation.

The compositions of the invention are intended for use for controlling a parasitic acarid infestation. The term "controlling a parasitic acarid infestation" refers to preventing, reducing or eliminating an infestation by such parasites on animals preferably by killing the insects and/ or acarids within hours or days.

The term "ectoparasite" refers to acarine and/or insect pests that commonly infest or infect animals. Examples of such ectoparasites include the egg, larval, pupal, nymphal and adult stages of mites, ticks, fleas, flies and lice.

In general, the composition according to the invention will contain an effective amount of the active ingredients, meaning a non-toxic but sufficient amount to provide the desired control effect. A person skilled in the art using routine experimentation may determine an appropriate "effective" amount in any individual case. Such an amount will depend on the age, condition, weight and type of the target animal.

Administration of the composition may be intermittent in time and may be administered daily, weekly, biweekly, monthly, bimonthly, quarterly, or even for longer durations of time. The time period between treatments depends upon factors such as the parasite(s) being treated, the degree of infestation, the type of mammal or bird and the environment where it resides. It is well within the skill level of the practitioner to determine a specific administration period for a particular situation. The treatment can, for example, be continuing or seasonal.

Preferably the treatment is carried out so as to administer to the animal a composition comprising a dose from 0.1 - 100 mg/ kg bodyweight and in particular from1-10 mg/ kg bodyweight of amitraz and a dose from 0.1 - 1000 mg/ kg bodyweight and in particular from 1 -100 mg/ kg bodyweight atipamezole.

It is further described herein a kit useful in the treatment of a parasite infestation of insects and/or acarids in an animal, which comprises amitraz and atipamezole in a veterinary acceptable formulation and instructions for the control of parasitic insect- and acarid- infestations on animals.

The compositions of the present invention may be prepared in a manner known per se.

In general the composition according to the current invention can be administered to all species of animals that have insect- or acarid- pest infestation. The recipient of the formulation may be a livestock animal, e.g. sheep, cattle, pig, goat or poultry; a laboratory test animal, e.g. guinea pig, rat or mouse; or a companion animal, e.g. dog, cat, rabbit, ferret or horse.

In another embodiment the invention relates to the use of the composition according to the invention for the manufacture of a veterinary medicament for the control of parasites, especially parasitic acarid- infestations on animals.

The experiments conducted in guinea-pigs demonstrated that the association showed a higher efficacy against ticks of *Rhipicephalus sp*., when compared to the efficacy of the separate compounds.

### Example

### Efficacy against Rhipicephalus sanguineus

Guinea pigs were infested with 100 nymphs of *Rhipicephalus sanguineus* (d0). Guinea pigs (n=3) were treated topically (bath treatment) with atipamezol (7.8 ppm), amitraz (2.0 ppm; 3.9 ppm) and two combinations of both (7.8 ppm atipamezol + 2.0 ppm amitraz; 7.8 ppm atipamezol + 3.9 ppm amitraz), respectively (d1). The control group remained untreated.

The number of detached ticks was assessed 1, 3, 6 and 24 hours after treatment (detachment effect).

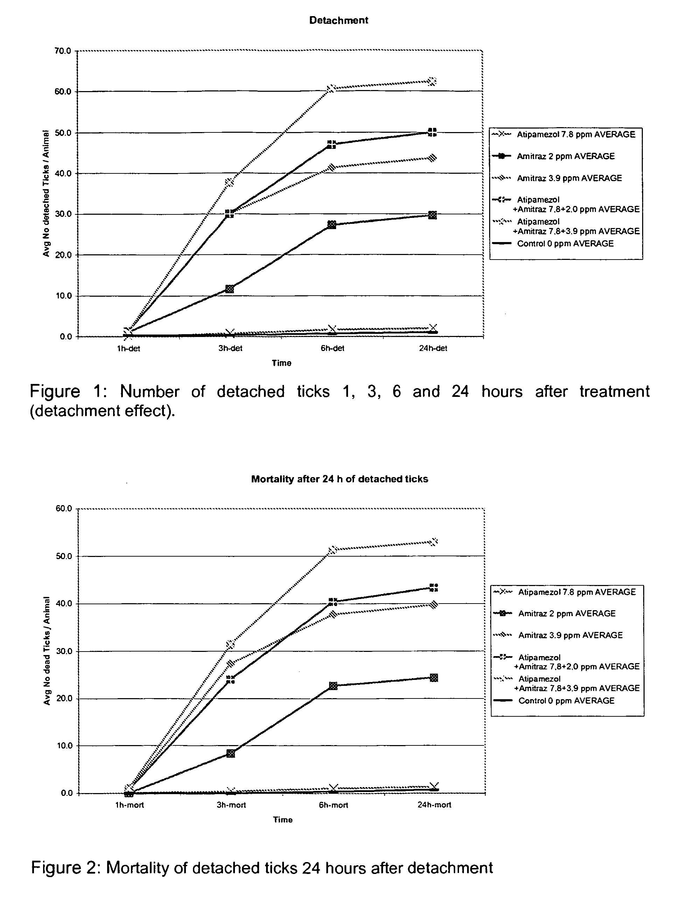

24 hours after treatment the average number of detached ticks per animal was 2.0 (7.8 ppm atipamezol), 29.7 (2.0 ppm amitraz), 43.7 (3.9 ppm amitraz), 50.0 (7.8 ppm atipamezol + 2.0 ppm amitraz), 62.3 (7.8 ppm atipamezol + 3.9 ppm amitraz) and 1.0 (untreated control). The results are illustrated in Figure 1.

The mortality of detached ticks was assessed 24 hours after detachment.

The average number of dead ticks after detachment was 1.3 (7.8 ppm atipamezol), 24.3 (2.0 ppm amitraz), 39.7 (3.9 ppm amitraz), 43.3 (7.8 ppm atipamezol + 2.0 ppm amitraz), 53.0 (7.8 ppm atipamezol + 3.9 ppm amitraz) and 0.7 (untreated control). The results are illustrated in Figure 2.

The average number of engorged ticks dropping per animal from d4 to d8 was assessed to determine the overall acaricidal efficacy compared to the control group.

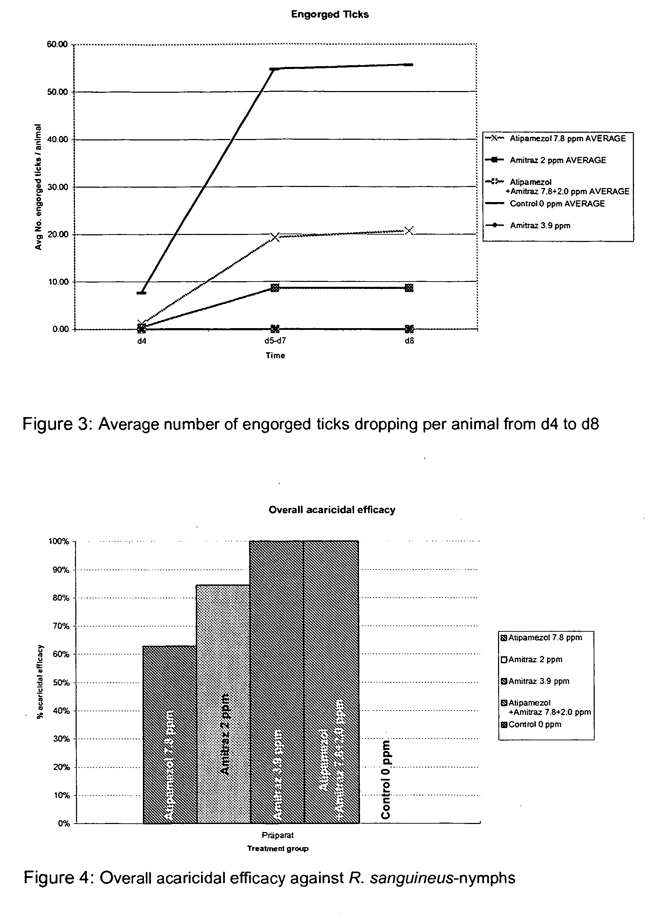

The average number of engorged ticks per animal was 20.7 (7.8 ppm atipamezol), 8.7 (2.0 ppm amitraz), 0 (3.9 ppm amitraz), 0 (7.8 ppm atipamezol + 2.0 ppm amitraz), 0 (7.8 ppm atipamezol + 3.9 ppm amitraz) and 55.7 (untreated control). The results are illustrated in Figure 3.

The overall acaricidal efficacy against *R. sanguineus*-nymphs was 63% (7.8 ppm atipamezol), 84% (2.0 ppm amitraz), 100% (3.9 ppm amitraz), 100% (7.8 ppm atipamezol + 2.0 ppm amitraz), 100% (7.8 ppm atipamezol + 3.9 ppm amitraz) and 0% (untreated control). The results are illustrated in Figure 4.

The treatments were well tolerated.

## Claims

1. Liquid veterinary composition comprising amitraz and atipamezole or its pharmaceutically acceptable salt.

2. Composition according to claim 1 **characterized that it** is in a form for topical administration to an animal.

3. Veterinary antiparasitic composition **characterized in that** the ratio of amitraz: atipamezole is between to 1:10.

4. Use of a composition according to claims 1 to 3 as active ingredients for the manufacture of a veterinary medicament for the control of ectoparasite infestations on animals.

5. Use according to claim 4 **characterized in that** the veterinary medicament comprises amitraz and atipamezole in a single dosage form.

6. Use according to claims 4 or 5 **characterized in that** amitraz is administered in a dose from 0.1 mg /kg bodyweight to 100mg / kg bodyweight and atipamezole in a dose from 0.1mg / kg bodyweight to 1000 mg / kg bodyweight.

## Patentansprüche

1. Flüssige tierärztliche Zusammensetzung, die Amitraz und Atipamezol oder ein pharmazeutisch unbedenkliches Salz davon umfasst.

2. Zusammensetzung nach Anspruch 1, **dadurch gekennzeichnet, dass** sie in einer Form für die topische Verabreichung an ein Tier vorliegt.

3. Tierärztliche antiparasitische Zusammensetzung, **dadurch gekennzeichnet, dass** das Verhältnis Amitraz:Atipamezol zwischen 8:1 und 1:10 liegt.

4. Verwendung einer Zusammensetzung nach den Ansprüchen 1 bis 3 als Wirkstoffe für die Herstellung eines tierärztlichen Arzneimittels für die Bekämpfung von Ektoparasitenbefall an Tieren.

5. Verwendung nach Anspruch 4, **dadurch gekennzeichnet, dass** das tierärztliche Arzneimittel Amitraz und Atipamezol in Einzeldosisform umfasst.

6. Verwendung nach Anspruch 4 oder 5, **dadurch gekennzeichnet, dass** Amitraz in einer Dosis von 0,1 mg/kg Körpergewicht bis 100 mg/kg Körpergewicht verabreicht wird und Atipamezol in einer Dosis von 0,1 mg/kg Körpergewicht bis 1000 mg/kg Körpergewicht verabreicht wird.

## Revendications

1. Composition vétérinaire liquide comprenant de l'amitraze et de l'atipamézole ou son sel pharmaceutiquement acceptable.

2. Composition selon la revendication 1, **caractérisée en ce qu'**elle est sous une forme destinée à l'administration par voie topique à un animal.

3. Composition antiparasitaire vétérinaire, **caractérisée en ce que** le rapport amitraze:atipamézole est compris entre 8:1 et 1:10.

4. Utilisation d'une composition selon les revendications 1 à 3, comme principes actifs pour la fabrication d'un médicament vétérinaire destiné à lutter contre les infestations ectoparasitaires sur les animaux.

5. Utilisation selon la revendication 4, **caractérisée en ce que** le médicament vétérinaire comprend l'amitraze et l'atipamézole sous une forme de dosage unitaire.

6. Utilisation selon la revendication 4 ou 5 **caractérisée en ce que** l'amitraze est administré dans une dose de 0,1 mg/kg de poids corporel à 100 mg/kg de poids corporel et l'atipamézole dans une dose de 0,1 mg/kg de poids corporel à 1000 mg/kg de poids corporel.
